Europäisches Patentamt

European Patent Office

Office européen des brevets

(11) Publication number: **0 106 202**
**B1**

# EUROPEAN PATENT SPECIFICATION

(45) Date of publication of patent specification: **22.02.89**

(51) Int. Cl.⁴: **A 61 K 9/66,** A 61 K 9/48, A 61 K 31/44

(21) Application number: **83109352.1**

(22) Date of filing: **20.09.83**

(54) **Soft capsule.**

(30) Priority: **20.09.82 JP 164706/82**

(43) Date of publication of application:
**25.04.84 Bulletin 84/17**

(45) Publication of the grant of the patent:
**22.02.89 Bulletin 89/08**

(84) Designated Contracting States:
**AT BE CH DE FR GB IT LI LU NL SE**

(56) References cited:
**AT-B- 244 505
DE-A-2 716 601
DE-A-2 903 778
DE-C- 844 640
FR-A-1 472 901
GB-A- 8 638
GB-A-1 341 121
GB-A-1 369 039
GB-A-1 507 521
US-A-3 061 517
US-A-3 456 051
US-A-3 992 215**

(73) Proprietor: **FUJISAWA PHARMACEUTICAL CO., LTD.**
**3, Doshomachi 4-chome Higashi-ku
Osaka-shi Osaka 541 (JP)**

(73) Proprietor: **Morishita Jintan Co., Ltd.**
**1-1-30, Tamatsukuri Higashi-ku
Osaka 540 (JP)**

(72) Inventor: **Hata, Takehisa**
**13-7, Nishikakiuchi Terado-cho
Mukou-shi Kyoto 617 (JP)**
Inventor: **Kanagawa, Nobuto**
**7-67, Aza Shijuso Kimokawa Inagawa-cho
Kawabe-gun Hyogo 666-02 (JP)**
Inventor: **Morishita, Takashi**
**c/o Morishita Jintan Co., Ltd. 1-1-30
Tamatsukuri
Higashi-ku Osaka 540 (JP)**
Inventor: **Suzuki, Toshiyuki**
**c/o Morishita Jintan Co., Ltd. 1-1-30,
Tamatsukuri
Higashi-ku Osaka 540 (JP)**

(74) Representative: **Bühling, Gerhard, Dipl.-Chem. et al**
**Patentanwaltsbüro Tiedtke-Bühling-Kinne
Grupe-Pellmann-Grams-Struif Bavariaring 4
D-8000 München 2 (DE)**

Courier Press, Leamington Spa, England.

EP 0 106 202 B1

(56) References cited:

K.H. WALLHÄUSSER, H. SCHMIDT,
"Sterilisation, Desinfektion, Konservierung,
Chemotherapie", 1967, GEORG THIEME
VERLAG, Stuttgart
E. BINDER et al., "Austria Codex 1981/82", 1981,

ÖSTERREICHISCHER APOTHEKER-VERLAG,
Wien, pp. 305, 843
M. NEGWER, "Organisch-Chemische
Arzneimittel und ihre Synonyme", 5th ed.,
1978, vol. 2, AKADEMIE-VERLAG, Berlin, p. 643

PATENT ABSTRACTS OF JAPAN, unexamined
applications, section C, vol. 6, no. 122, July 7,
1982, THE PATENT OFFICE JAPANESE
GOVERNMENT, p. 50 C 112

P.H. LIST, L. HÖRHAMMER, Hagers Handbuch
der pharmazeutischen Praxis, 4th ed., vol. 7,
Part A, 1971, SPRINGER-VERLAG, Berlin,
Heidelberg, New York, pp. 482-491

# Description

This invention relates to a soft capsule which is prepared by enclosing an oily substance with a film containing a water-soluble medicinal substance.

The object of this invention is to provide a soft capsule which can be applied to a water-soluble medicinal substance.

It has been known to prepare soft capsules by enclosing oily substances containing medicinal substances with a gelatin film.

When the medicinal substance is water-soluble a soft capsule has been prepared by enclosing a suspension of the water-soluble medicinal substance in oil with a gelatin film.

According to this method, however, it is inevitable that the quality of the capsules deteriorates in respect of content of the medicinal substance in each capsule because it is difficult to maintain an even amount of suspension to be filled in each capsule during the preparation.

This tendency becomes more and more noticeable with the decrease of the content of the medicinal substance, which sometimes results in serious faults in the quality of medicine.

FR—A—1 472 901 discloses a capsule containing a filling composition the shell of which consists of a gelatin film containing vitamin $B_{12}$. The filling composition contains other vitamins in an oily substance. However, the content of vitamin $B_{12}$ in the film is quite small (0,5 to 10 μg per capsule), that is to say, the ratio of vitamin $B_{12}$ in the film of the capsule of the citation seems to be about 0,005 to 0,1%. This small amount does not effect the strength of the film.

US—A—3 456 051 discloses a soft capsule, wherein the drug is contained in the inner oil, while the soft gelatin, however, contains glycine. Glycine hydrochloride is not a drug but a buffer agent, as disclosed in column 3, line 50.

GB—A—1 369 039 discloses in example 20 hydroxypropyl cellulose containing 2% sodium dioctylsulphosuccinate as shell composition. Sodium dioctylsulphosuccinate is not a medicinal substance but a surfactant as can be gathered from page 2, line 88.

According to DE—A—844 640 the gelatin capsule consists of a preferably uncured gelatin composition which can be easily dissolved in the stomach or in the upper portion of the intestines. A liquid medical substance, especially a vermifuge, is contained in the capsule. An uncured gelatin composition is not a soft gelatin capsule but a conventional hard gelatin capsule.

DE—A—2 903 778 discloses soft gelatin capsules containing medicinal substance in the film. This citation is directed to medicines which can be administered buccally or sublingually. As stated on pages 4 and 5 the previous known soft gelatin capsules have drawbacks which can be avoided when such soft capsules are coated with polymers in order to prevent the deformation of the capsules at higher temperatures. Accordingly, there are examples relating to the polymer used for coating said capsule but there are no examples relating to the soft capsule containing medicinal substance in the film per se.

US—A—3 061 517 and GB—A—1 341 121 disclose the formulation of maleic acid chlorpheniramine in a soft capsule but this medicinal substance is not contained in the film of the soft capsule per se.

The inventors have attempted to resolve this kind of problem, and finally got the idea to add the water-soluble medicinal substance in the film. Such an idea could be obtained by going against the ordinary knowledge that medicinal substance is contained in an oily phase of a soft capsule. The soft capsule of this invention has a different constitution from the conventional soft capsule defined in pharmacopoeia.

The soft capsule of this invention is prepared by enclosing an oily substance selected from tricaprylic acid glyceride and sesame oil, with a film containing about 1,5 mg of sodium picosulfate.

The main component of the film is not restricted, but usually the film consists of gelatin optionally treated with organic or inorganic acid or alkaline substance. Conventional additives to the film such as coloring agent, plasticizer, antiseptic, wetting agent, solubilizing agent, disintegrant, relishes and antioxidizing agents, can be optionally added to the film component.

This invention is applied to the water-soluble sodium pico sulfate which can be dissolved in the film component. When the sodium pico sulfate can not be easily dissolved in the film component, a solubilizing agent can optionally be used. The content of the sodium pico sulfate contained in the film is about 1,5 mg.

The oily substance to be enclosed by the film as mentioned above is selected from tricaprylic acid glyceride and sesame oil.

These oily substances can optionally contain a medicinal substance which can be dissolved therein.

The soft capsule of this invention can be prepared by conventional method, and preferably by the method described in the specification of Japanese Patent Publication No. 1067/1978, according to which the soft capsule equalized in the weight of the film can be easily prepared.

The soft capsule of this invention possesses superior effect as follows. That is, the content of the sodium picosulfate in each capsule can be easily equalized even though this medicinal substance is water-soluble. And further, the sodium picosulfate can be dissolved in a short time after administration, since this substance is contained in the surface part of the soft capsule.

In addition to the above, beautiful soft capsule can be obtained when both of the oily substance and the film are transparent because the medicinal substance is not suspended in the oily substance but the substance is dissolved in the film component.

The method for preparing the soft capsule of this invention is explained in more detail in the following Examples.

Example 1
(Film components)

| | |
|---|---|
| Sodium picosulfate | 1.5 mg |
| Gelatin | 7 mg |
| D-Sorbitol | 1 mg |
| Glycerol | 2 mg |
| Ethylparaben | 0.03 mg |
| Propylparaben | 0.01 mg |
| Reddish coloring agent | q.s. |

(Oily Substance)

| | |
|---|---|
| Tricaprylic acid glyceride | 23 mg |

Soft capsule with a diameter of 4 mm were prepared by the method described in the specification of Japanese Patent Publication No. 1067/1978 by using the film components and the oily substance in the ratio as described in the above.

Example 2

Soft capsules were prepared by the method described in the specification of Japanese Patent Publication No. 1067/1978 by using sesame oil instead of tricaprylic acid glyceride in Example 1.

**Claims**

1. A soft capsule which is prepared by enclosing an oily substance selected from tricaprylic acid glyceride and sesame oil, with a film containing about 1.5 mg of sodium picosulfate.

2. A soft capsule according to claim 1, wherein the main component of the film is gelatin.

**Patentansprüche**

1. Weichkapsel, die hergestellt wird, indem man eine ölige Substanz, die aus Glycerintricaprylat und Sesamöl ausgewählt ist, mit einem Film, der etwa 1,5 mg Natriumpicosulfat enthält, umhüllt.

2. Weichkapsel nach Anspruch 1, bei der der Hauptbestandteil des Filmes Gelatine ist.

**Revendications**

1. Capsule molle qui est préparée en enfermant une substance huileuse choisie parmi la glycéride d'acide tricaprylique et l'huile de sésame, avec une pellicule contenant environ 1,5 mg de picosulfate de sodium.

2. Capsule molle selon la revendication 1, dans laquelle le composant principal de la pellicule est la gélatine.